(19) Europäisches Patentamt
European Patent Office
Office européen des brevets

(11) Veröffentlichungsnummer: **0 160 219**
**A1**

# (12) EUROPÄISCHE PATENTANMELDUNG

(21) Anmeldenummer: **85103859.6**

(22) Anmeldetag: **30.03.85**

(51) Int. Cl.⁴: **C 07 D 251/16**
C 07 D 251/46, C 07 D 251/22
C 07 D 239/42, C 07 D 239/47
C 07 D 403/12, C 07 D 405/12
C 07 D 413/12, A 01 N 47/34
A 01 N 47/36

(30) Priorität: **11.04.84 DE 3413565**

(43) Veröffentlichungstag der Anmeldung:
**06.11.85 Patentblatt 85/45**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Stetter, Jörg, Dr.**
**Gellertweg 4**
**D-5600 Wuppertal 1(DE)**

(72) Erfinder: **Wroblowsky, Heinz-Jürgen, Dr.**
**Gladbacher Strasse 36**
**D-4018 Langenfeld(DE)**

(72) Erfinder: **Eue, Ludwig, Dr.**
**Paul-Klee-Strasse 36**
**D-5090 Leverkusen 1(DE)**

(72) Erfinder: **Schmidt, Robert R., Dr.**
**Im Waldwinkel 110**
**D-5060 Bergisch-Gladbach 2(DE)**

(72) Erfinder: **Santel, Hans-Joachim, Dr.**
**Gerstenkamp 19**
**D-5000 Köln 80(DE)**

(72) Erfinder: **Lürssen, Klaus, Dr.**
**August-Kierspel-Strasse 151**
**D-5060 Bergisch-Gladbach 2(DE)**

(72) Erfinder: **Hänssler, Gerd, Dr.**
**Heymannstrasse 40**
**D-5090 Leverkusen 1(DE)**

(54) **Substituierte Sulfonylharnstoffe.**

(57) Die Erfindung betrifft neue substituierte Sulfonylharnstoffe der allgemeinen Formel (I)

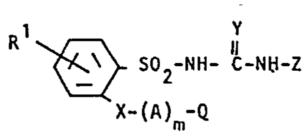

(worin die Variablen $R^1$, A, m, Q, X, Y und Z die in der Beschreibung angegebenen Bedeutungen haben), Verfahren zu ihrer Herstellung und ihre Verwendung als Pflanzenbehandlungsmittel, insbesondere als Herbizide, Pflanzenwachstumsregulatoren und Fungizide.

EP 0 160 219 A1

BAYER AKTIENGESELLSCHAFT 5090 Leverkusen, Bayerwerk

Konzernverwaltung RP
Patentabteilung Bi/Ke

Ib

## Substituierte Sulfonylharnstoffe

Die Erfindung betrifft neue substituierte Sulfonylharnstoffe, mehrere Verfahren zu ihrer Herstellung und ihre Verwendung als Pflanzenschutzmittel, insbesondere als Herbizide, Pflanzenwachstumsregulatoren und Fungizide.

Es ist bereits bekannt, daß bestimmte substituierte Sulfonylharnstoffe, wie beispielsweise der N-(2-Cyanmethoxyphenyl-sulfonyl)-N'-(4-methoxy-6-methyl-pyrimidin-2-yl)-harnstoff oder der N-(2-Cyanmethoxyphenylsulfonyl)-N'-(4,6-dimethyl-pyrimidin-2-yl)-harnstoff,herbizide und pflanzenwuchsregulierende Eigenschaften besitzen (vgl. EP 85 028).

Die herbizide und pflanzenwachstumsregulierende Wirkung dieser vorbekannten substituierten Sulfonylharnstoffe ebenso wie deren Verträglichkeit gegenüber wichtigen Kulturpflanzen ist jedoch nicht immer in allen Anwendungsbereichen völlig zufriedenstellend. Über eine fungizide Wirkung der bekannten Sulfonylharnstoffe ist bisher nichts bekannt.

Es wurden neue substituierte Sulfonylharnstoffe der allgemeinen Formel (I),

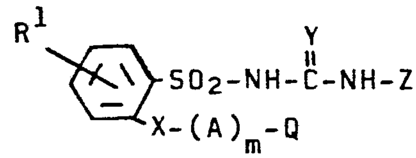 (I)

in welcher

$R^1$ für Wasserstoff, Halogen, Nitro, Alkyl, Alkoxy, Alkoxycarbonyl oder Halogenalkyl steht,

X für Sauerstoff, Schwefel, die Sulfinyl- oder die Sulfonylgruppe steht,

Y für Sauerstoff oder Schwefel steht,

Z für den Rest 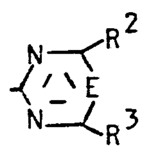 steht,

wobei E für Stickstoff oder die CH-Gruppe steht und
$R^2$ und $R^3$ jeweils unabhängig voneinander für Alkyl, Alkoxy oder Alkoxyalkyl stehen,

A für einen zweifach verknüpften Alkylen- oder Alkenylenrest steht,

m für 0 oder 1 steht und

Q für Pivaloyl, für Methoximinoalkyl, für einen der Heterocyclen der Formel

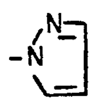 , 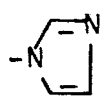 , 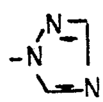 , 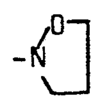 , 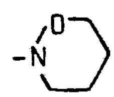 oder 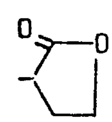

oder für einen der jeweils gegebenenfalls substituierten und/oder gegebenenfalls teilhydrierten C-verknüpften Heterocylen der Formel

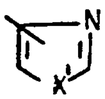 , 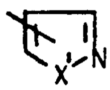 , 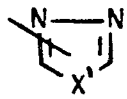 oder 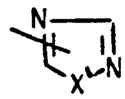 steht

wobei X' jeweils für Sauerstoff, Schwefel oder eine NH-Gruppe steht,

außerdem für Acetyl steht, wenn $R^2$ und $R^3$ gleichzeitig für Alkyl stehen und weiterhinfür 1,1-Dichlorcycloprop-2-yl steht, wenn E für die CH-Gruppe steht,

gefunden.

Weiterhin wurde gefunden, daß man die neuen substituierten Sulfonylharnstoffe der allgemeinen Formel (I) erhält, wenn man

(a) heterocyclische Aminoverbindungen der allgemeinen Formel (II),

$$H_2N-Z \qquad (II)$$

in welcher

Z die oben angegebene Bedeutung hat,

entweder

α) mit substituierten Sulfonyliso-(thio-)cyanaten der allgemeinen Formel (III)

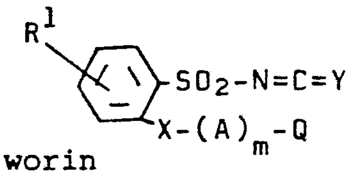

(III)

worin

$R^1$, X, Y, A, Q und m die oben angegebenen Bedeutungen haben,

oder

ß) mit N-Sulfonyl-(thio-)-carbamaten der allgemeinen Formel (IV),

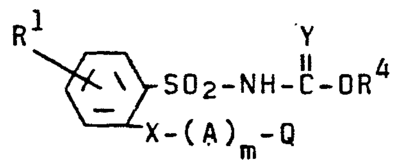

(IV)

in welcher

$R^1$, X, Y, A, Q und m die oben angegebene Bedeutung haben und

$R^4$ für Alkyl oder Aryl steht,

jeweils gegebenenfalls in Gegenwart eines Verdünnungsmittels und jeweils gegebenenfalls in Gegenwart eines Säurebindemittels umsetzt, oder wenn man

(b) substituierte Sulfonamide der allgemeinen Formel (V),

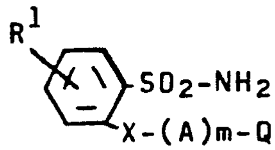

(V)

in welcher

$R^1$, X, A, Q und m die oben angegebene Bedeutung haben,

entweder

α) mit Iso-(thio)-cyanaten der Formel (VI),

$$Y=C=N-Z \qquad (VI)$$

in welcher

Y und Z die oben angegebene Bedeutung haben

oder

β) mit (Thio)-Carbamaten der Formel (VII),

$$R^{4'}-O-\overset{\overset{\displaystyle Y}{\|}}{C}-NH-Z \qquad (VII)$$

in welcher

Y und Z die oben angegebene Bedeutung haben und

$R^{4'}$ für Alkyl oder Aryl steht,

jeweils gegebenenfalls in Gegenwart eines Verdünnungsmittels und jeweils gegebenenfalls in Gegenwart eines Säurebindemittels umsetzt.

Schließlich wurde gefunden, daß die neuen substituierten Sulfonylharnstoffe der Formel (I) herbizide, insbesondere auch selektiv-herbizide und pflanzenwachstumsregulatorische Eigenschaften besitzen.

Ueberraschenderweise besitzen die neuen substituierten Sulfonylharnstoffe der Formel (I) neben einer besseren herbiziden Wirkung gegen wichtige Schadpflanzen auch eine verbesserte Verträglichkeit gegenüber wichtigen Kulturpflanzen und ebenfalls verbesserte pflanzenwachtumsregulatorische Eigenschaften im Vergleich zu den aus dem Stand der Technik bekannten substituierten Sulfonylharnstoffen N-(2-Cyanmethoxy-phenylsulfonyl)-N'-(4-methoxy-6-methyl-pyrimidin-2-yl)harnstoff bzw. N-(2-Cyanmethoxy-phenylsulfonyl)-N'-(4,6-dimethyl-pyrimidin-2-yl)-harnstoff, welche chemisch und wirkungsmäßig naheliegende Verbindungen sind. Darüber hinaus besitzen die neuen substituierten Sulfonylharnstoffe überraschenderweise auch fungizide Eigenschaften, und zwar bessere fungizide Eigenschaften als aus dem Stand der Technik bekannte Fungizide, wie beispielsweise das Zinkethylen-1,2-bis-(dithiocarbamat), welches eine wirkungsmäßig naheliegende Verbindung ist.

Die neuen erfindungsgemäßen substituierten Sulfonylharnstoffe sind durch die Formel (I) allgemein definiert. Bevorzugt sind Verbindungen der Formel (I), bei welchen

$R^1$ für Wasserstoff, Fluor, Chlor, Brom, Jod, Nitro, jeweils geradkettiges oder verzweigtes Alkyl, Alkoxy oder Alkoxycarbonyl mit jeweils bis zu 4 Kohlenstoffatomen in den einzelnen Alkylteilen oder für geradkettiges oder verzweigtes Halogenalkyl mit bis zu 4 Kohlenstoffatomen und bis zu 9 gleichen oder verschiedenen Halogenatomen steht,

X für Sauerstoff, Schwefel, die Sulfinyl- oder die Sulfonylgruppe steht,

Y für Sauerstoff oder Schwefel steht,

Z für den Rest 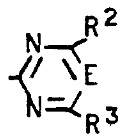 steht,

wobei E für Stickstoff oder die CH-Gruppe steht und $R^2$ und $R^3$ jeweils unabhängig voneinander für jeweils geradkettiges oder verzweigtes Alkyl, Alkoxy oder Alkoxyalkyl mit bis zu 4 Kohlenstoffatomen in den einzelnen Alkylteilen stehen,

A für einen jeweils geradkettigen oder verzweigten zweifach verknüpften Alkylen- oder Alkenylenrest mit jeweils bis zu 4 Kohlenstoffatomen steht,

m für 0 oder 1 steht und

Q für Pivaloyl, für geradkettiges oder verzweigtes Methoximinoalkyl mit bis zu 4 Kohlenstoffatomen im Alkylteil, für einen der Heterocyclen der Formel

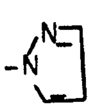 , 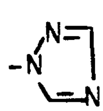 , 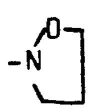 , 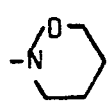 , 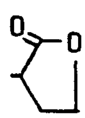 oder 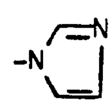

oder für einen der jeweils gegebenenfalls einfach oder mehrfach, gleich oder verschieden durch Halogen, Cyano, geradkettiges oder verzweigtes Alkyl oder Halogenalkyl mit jeweils bis zu 4 Kohlenstoffatomen und gegebenenfalls bis zu 9 gleichen oder verschiedenen Halogenatomen substituierten und/oder gegebenenfalls teilhydrierten C-verknüpften Heterocyclen der Formel

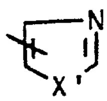 ; 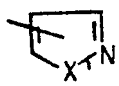 ; 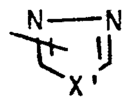 ; 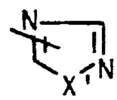 steht,

wobei X' jeweils für Sauerstoff, Schwefel oder die NH-Gruppe steht,

außerdem für Acetyl steht, wenn $R^2$ und $R^3$ gleichzeitig für Alkyl stehen und weiterhin für 1,1-Dichlorcycloprop-2-yl steht, wenn E für die CH-Gruppe steht.

Besonders bevorzugt sind substituierte Sulfonylharnstoffe der Formel (I), bei welchen

$R^1$ für Wasserstoff, Fluor, Chlor, Brom, Nitro, Methyl, Ethyl, Methoxy, Ethoxy, Methoxycarbonyl, Ethoxycarbonyl, Trifluorethyl oder Trichlorethyl steht,

X für Sauerstoff, Schwefel, die Sulfinyl- oder die Sulfonylgruppe steht,

Y für Sauerstoff oder Schwefel steht,

Z für den Rest 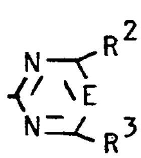 steht, wobei

E für Stickstoff oder die CH-Gruppe steht und

$R^2$ und $R^3$ unabängig voneinander für Methyl, Methoxy oder Methoxymethyl stehen,

A für $-CH_2-$; $-CH_2-CH_2-$; $-\overset{CH_3}{C}H-$; $-\overset{CH_3}{C}H-CH_2-$; $-CH=CH-$; $-CH_2-CH_2-CH_2-$ oder $-\overset{CH_3}{C}H-CH_2-CH_2-$ steht,

m für 0 oder 1 steht und

Q für Pivaloyl, 1-Methoximinoethyl, 1-Methoximinopropyl, 1-Methoximino-2-methylpropyl, für einen der Heterocyclen

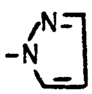 , 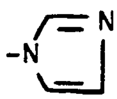 , 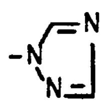 , 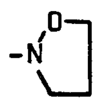 , 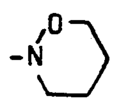 oder 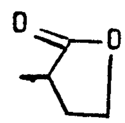

oder für einen jeweils gegebenenfalls einfach oder zweifach, gleich oder verschieden durch Methyl, Ethyl, Chlor, Brom, Cyano, Trifluormethyl, Trichlormethyl, Dichlorfluormethyl, Difluorchlormethyl, Chlormethyl oder Dichlormethyl substituierten Heterocyclus der Formel

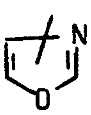 ; 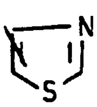 ; 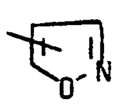 ; 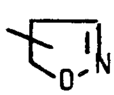 ; 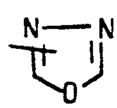 ; 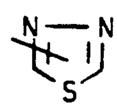 ;

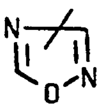 ; 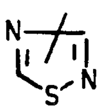 ; 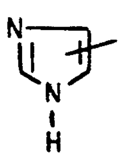 ; 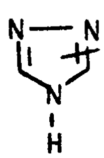 oder 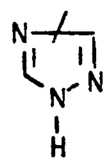 steht,

außerdem für Acetyl steht, wenn $R^2$ und $R^3$ gleichzeitig für Methyl stehen und weiterhin für 1,1-Dichlorcycloprop-2-yl steht, wenn E für die CH-Gruppe steht.

Im einzelnen seien außer den bei den Herstellungsbeispielen genannten Verbindungen die folgenden Verbindungen der Formel (I) genannt:

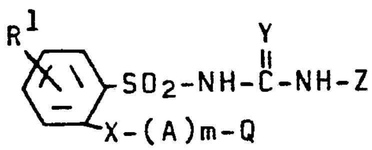

(I)

Tabelle 1

| $R^1$ | X | -(A)m- | Q | Y | Z |
|---|---|---|---|---|---|
| H | O | $-CH_2-$ | 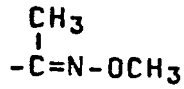 | S | 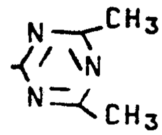 |
| H | O | $-CH_2-$ | 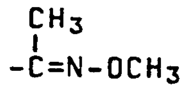 | S | 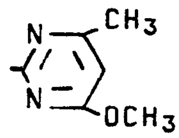 |
| H | O | $-CH_2-$ | 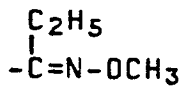 | O | 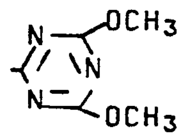 |
| H | O | $-CH_2-$ | 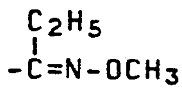 | O | 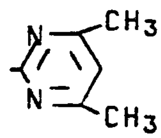 |
| H | O | $-CH_2-$ | 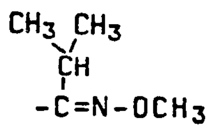 | O | 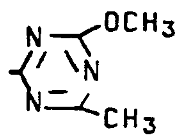 |
| H | O | $-CH_2-$ | 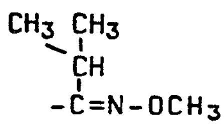 | O | 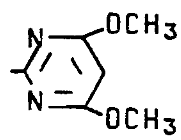 |
| H | S | $-CH_2-$ | 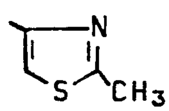 | O | 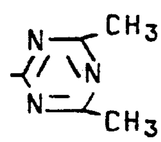 |
| H | S | $-CH_2-$ | 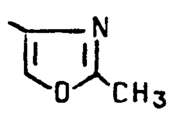 | O | 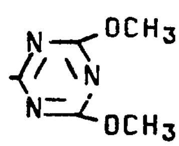 |

Tabelle 1 (Fortsetzung)

| $R^1$ | X | -(A)m- | Q | Y | Z |
|---|---|---|---|---|---|
| H | S | $-CH_2-$ | 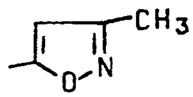 | O | 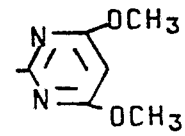 |
| H | S | $-CH_2-$ | 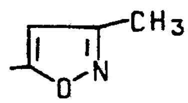 | O | 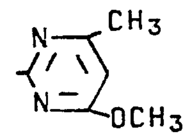 |
| H | O | $-CH_2-$ | $(CH_3)_3C-CO-$ | S | 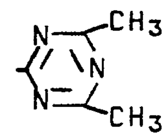 |
| H | O | $-CH_2-$ | $(CH_3)_3C-CO-$ | S | 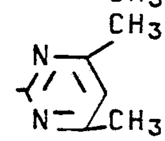 |
| H | S | $-CH_2-$ | $(CH_3)_3C-CO-$ | S | 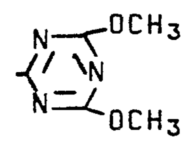 |
| H | S | $-CH_2-$ | $(CH_3)C-CO-$ | S | 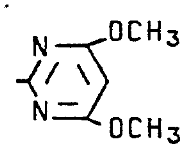 |
| H | O | - | 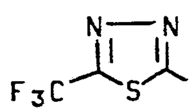 | O | 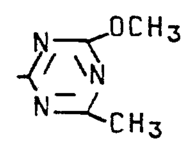 |
| H | O | - | 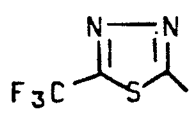 | S | 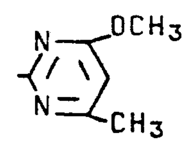 |
| H | O | - | 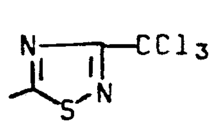 | O | 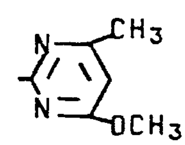 |

Tabelle 1 (Fortsetzung)

| $R^1$ | X | -(A)m- | Q | Y | Z |
|---|---|---|---|---|---|
| H | O | - | 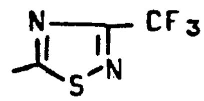 | O | 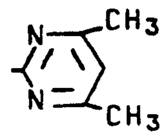 |
| H | O | $-CH_2-$ | 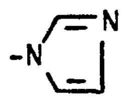 | O | 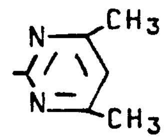 |
| H | O | $-CH_2-$ | 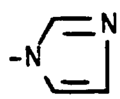 | O | 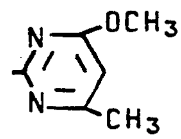 |
| H | O | $-CH_2-$ | 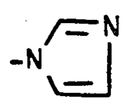 | O | 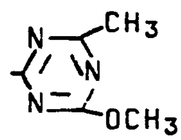 |
| H | $SO_2$ | - | 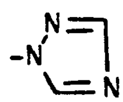 | O | 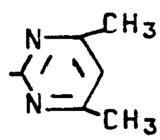 |
| H | $SO_2$ | - | 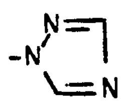 | O | 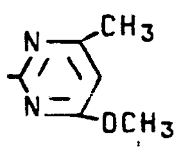 |
| H | $SO_2$ | - | 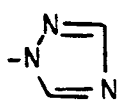 | O | 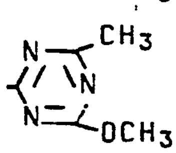 |
| H | $SO_2$ | - | 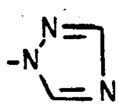 | O | 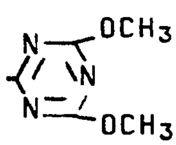 |
| H | $SO_2$ | $-CH_2-$ | $-\underset{CH_3}{C}=N-OCH_3$ | O | 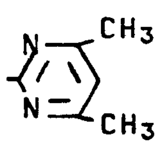 |

Tabelle 1 (Fortsetzung)

| $R^1$ | X | -(A)m- | Q | Y | Z |
|---|---|---|---|---|---|
| H | $SO_2$ | $-CH_2-$ | $-\underset{CH_3}{\underset{\mid}{C}}=N-OCH_3$ | O | 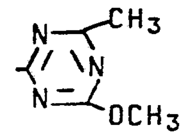 |
| H | $SO_2$ | $-CH_2-$ | $-\underset{CH_3}{\underset{\mid}{C}}=N-OCH_3$ | O | 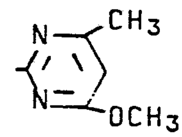 |
| H | $SO_2$ | $-CH_2-$ | $-\underset{CH_3}{\underset{\mid}{C}}=N-OCH_3$ | O | 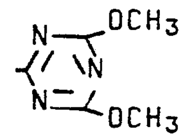 |
| H | $SO_2$ | $-\overset{CH_3}{\overset{\mid}{C}}H-$ | $-\underset{CH_3}{\underset{\mid}{C}}=N-OCH_3$ | O | 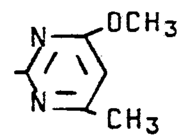 |
| H | $SO_2$ | $-\overset{CH_3}{\overset{\mid}{C}}H-$ | $-\underset{CH_3}{\underset{\mid}{C}}=N-OCH_3$ | O | 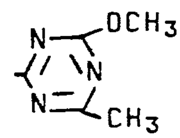 |
| H | S | $-CH_2-$ | $-\underset{CH_3}{\underset{\mid}{C}}=N-OCH_3$ | O | 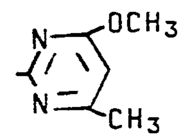 |
| H | S | $-CH_2-$ | $-\underset{CH_3}{\underset{\mid}{C}}=N-OCH_3$ | O | 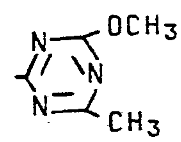 |
| H | $SO_2$ | - | 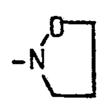 | O | 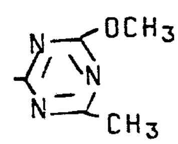 |
| H | $SO_2$ | - | 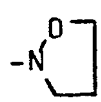 | O | 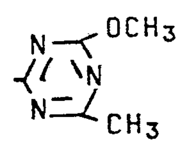 |

Tabelle 1 (Fortsetzung)

| $R^1$ | X | -(A)m- | Q | Y | Z |
|---|---|---|---|---|---|
| H | $SO_2$ | - | 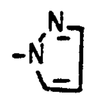 | O | 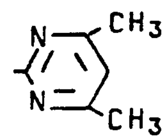 |
| H | $SO_2$ | - | 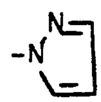 | O | 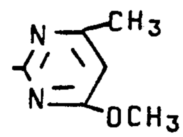 |
| H | $SO_2$ | - | 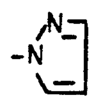 | O | 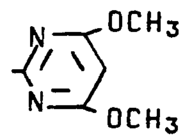 |
| H | $SO_2$ | - | 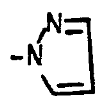 | O | 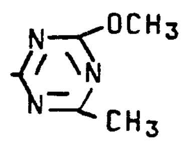 |
| H | $SO_2$ | - | 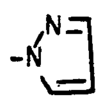 | O | 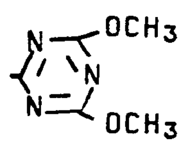 |
| H | $SO_2$ | - | 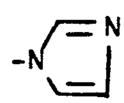 | O | 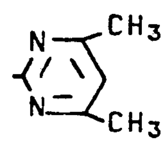 |
| H | $SO_2$ | - | 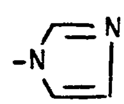 | O | 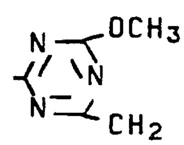 |
| H | $SO_2$ | - | 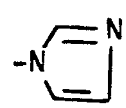 | O | 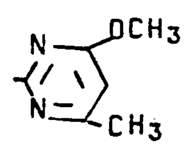 |
| H | $SO_2$ | - | 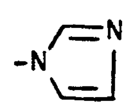 | O | 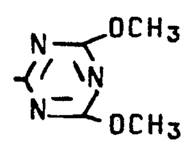 |

Verwendet man als Ausgangsstoffe beispielsweise 2-Amino-4,6-dimethylpyrimidin und 2-(2-Methyl-1,3-thiazol-5-ylmethoxy)-benzolsulfonylisocyanat, so läßt sich der Reaktionsablauf des erfindungsgemäßen Verfahrens (a - α) durch das folgende Formelschema darstellen:

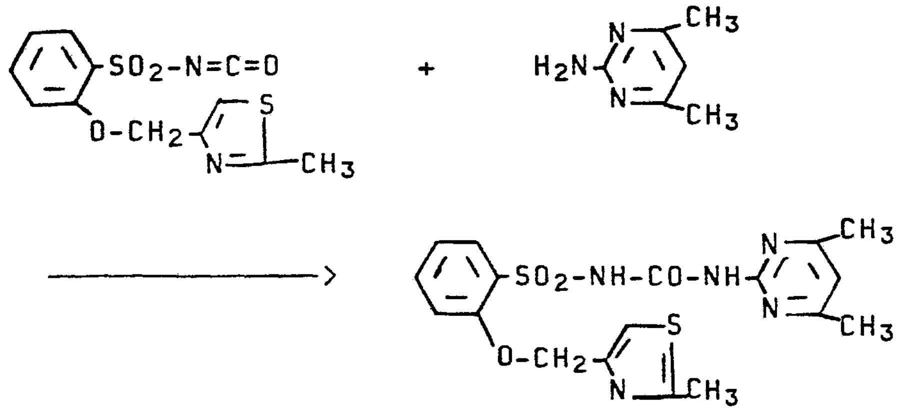

Verwendet man als Ausgangsstoffe beispielsweise 2-Amino-4-methoxy-6-methyl-1,3,5-triazin und N-[(2-Isoxazol-5-ylmethoxy)-phenylsulfonyl]-O-phenyl-carbamat, so läßt sich der Reaktionsablauf des erfindungsgemäßen Verfahrens (a - ß) durch das folgende Formelschema darstellen:

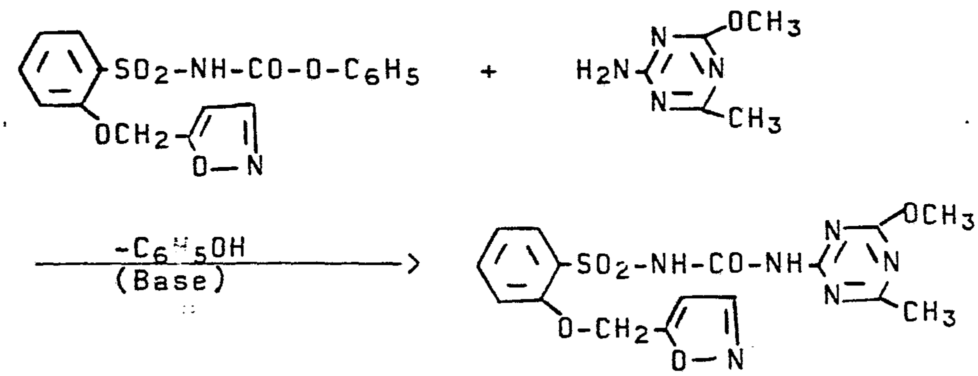


Verwendet man als Ausgangsstoffe beispielsweise 2-(5-Methyl-1,3,4-oxadiazol-2-ylmethoxy)-benzolsulfonamid und 4,6-Dimethoxypyrimidin-2-yl-isocyanat, so läßt sich der Reaktionsablauf des erfindungsgemäßen Verfahrens (b - α) durch das folgende Formelschema darstellen:

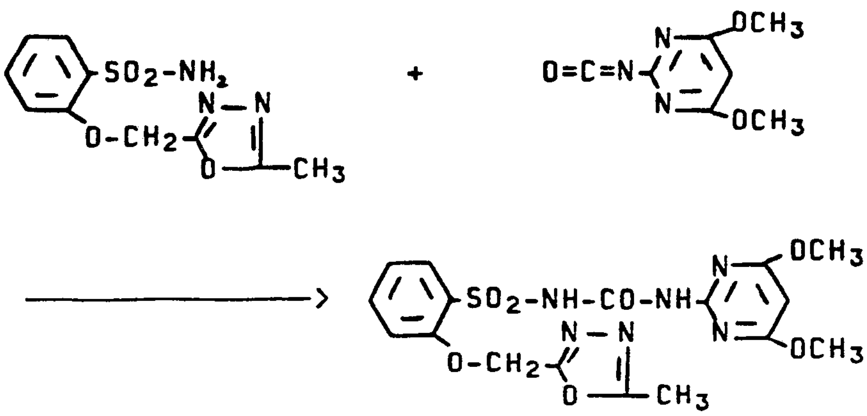

Verwendet man als Ausgangsstoffe beispielsweise 2-(3-Methyl-isoxazol-5-ylmethoxy)-benzolsulfonamid und N-(4,6-Dimethyl-1,3,5-triazin-2-yl)-O-phenyl-carbamat, so läßt sich der Reaktionsablauf des erfindungsgemäßen Verfahrens (b - ß) durch das folgende Formelschema darstellen:

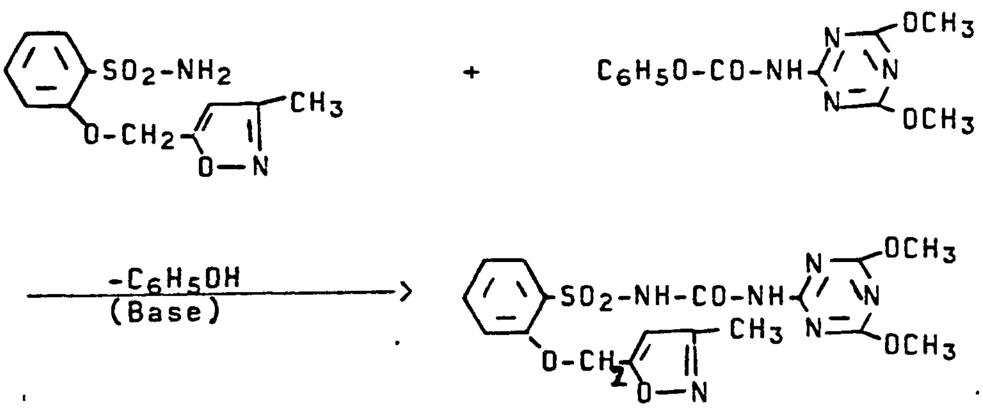


Die zur Durchführung des erfindungsgemäßen Verfahrens (a) als Ausgangsstoffe benötigten heterocyclischen Aminoverbindungen sind durch die Formel (II) allgemein definiert. In dieser Formel (II) steht Z vorzugsweise für diejenigen Reste, die schon bei der Beschreibung der erfindungsgemäßen Stoffe der Formel (I) als bevorzugt für diesen Substituenten angegeben wurden.

Die heterocyclischen Aminoverbindungen der Formel (II) sind bekannt (vergl. z. B. EP 73 627, EP 73 562, EP 76 637,

US-PS 4 127 405).

Die zur Durchführung des erfindungsgemäßen Verfahrens (a - α) weiterhin als Ausgangsstoffe benötigten Sulfonyliso-(thio)-cyanate sind durch die Formel (III) allgemein definiert. In dieser Formel (III) stehen $R^1$, X, Y, A, Q und m vorzugsweise für diejenigen Reste und Zahlen, die schon bei der Beschreibung der erfindungsgemäßen Stoffe der Formel (I) als bevorzugt für diese Substituenten und Indizes genannt wurden.

Die Sulfonyliso-(thio-)cyanate der Formel (III) sind noch nicht bekannt.

Man erhält sie, wenn man substituierte Sulfonamide der Formel (V),

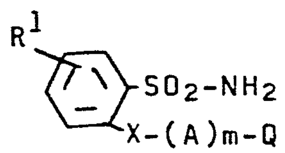

(V)

in welcher

$R^1$, X, A, Q und m die oben angegebene Bedeutung haben,

mit Phosgen oder Thiophosgen, gegebenenfalls in Gegenwart eines Verdünnungsmittels wie beispielsweise Chloroform und gegebenenfalls in Gegenwart eines Säurebindemittels wie beispielsweise Triethylamin bei Temperaturen zwischen -30 °C und +50 °C umsetzt.

Die zur Durchführung des erfindungsgemäßen Verfahrens (a - β) weiterhin als Ausgangsstoffe benötigten N-Sulfonyl-(thio-)carbamate sind durch die Formel (IV) allgemein definiert.

In dieser Formel (IV) stehen $R^1$, X, Y, A, Q und m vorzugsweise für diejenigen Reste und Indizes, die schon bei der Beschreibung der erfindungsgemäßen Stoffe der Formel (I) als bevorzugt für diese Substituenten und Indizes angegeben wurden. $R^4$ steht vorzusweise für Methyl, Ethyl oder Phenyl.

Die N-Sulfonyl-(thio-)carbamate der Formel (IV) sind noch nicht bekannt. Man erhält sie, wenn man substituierte Sulfonamide der Formel (V),

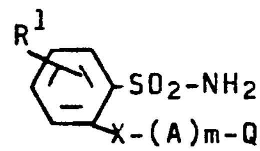

(V)

in welcher

$R^1$, X, A, Q und m  die oben angegebene Bedeutung haben,

mit (Thio-)Carbonaten oder Halogen(-thio)ameisensäureestern der Formel (VIII),

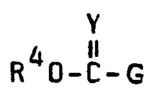

(VIII)

in welcher

$R^4$ und Y  die oben angegebene Bedeutung haben und

G  für einen Rest $R^4$-O- oder Halogen, insbesondere für Chlor, Methoxy, Ethoxy oder Phenoxy steht,

gegebenenfalls in Gegenwart eines Verdünnungsmittels wie beispielsweise Dimethylformamid und gegebenenfalls in Gegenwart eines Säurebindemittels wie beispielsweise Natriumhydrid oder Diazabicycloundecen (DBU) bei Temperaturen

zwischen -20 °C und +30 °C umsetzt.

Die zur Durchführung des erfindungsgemäßen Verfahrens (b) sowie zur Herstellung der Vorprodukte der Formeln (III) und (IV) als Ausgangsstoffe benötigten substituierten Sulfonamide sind durch die Formel (V) allgemein definiert. In dieser Formel (V) stehen $R^1$, A, Q, X und m vorzugsweise für diejenigen Reste und Indizes, die schon bei der Beschreibung der erfindungsgemäßen Stoffe der Formel (I) als bevorzugt für diese Substituenten oder Indizes genannt wurden.

Die substituierten Sulfonamide der Formel (V) sind noch nicht bekannt.

Man erhält sie, wenn man Sulfonamide der Formel (IX),

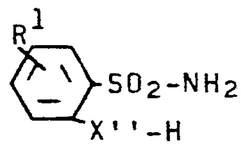

(IX)

in welcher

$R^1$ die oben angegebene Bedeutung hat und

X'' für Sauerstoff oder Schwefel steht,

mit Halogeniden der Formel (X),

Hal-(A)m-Q (X)

in welcher

A, Q und m die oben angegebene Bedeutung haben,

gegebenenfalls in Gegenwart eines Verdünnungsmittels, wie beispielsweise Acetonitril und gegebenenfalls in Gegenwart eines Säurebindemittels wie beispielsweise Kaliumcarbonat bei Temperaturen zwischen 20 °C und 120 °C umsetzt, und gegebenenfalls die so erhältlichen Sulfonamide der Formel (Va),

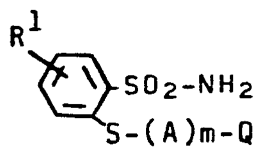

(Va)

in welcher

$R^1$, A, Q und m die oben angegebene Bedeutung haben,

in einer 2. Stufe beispielsweise mit Oxidationsmitteln der Formel (XI)

$$H\text{-}O\text{-}O\text{-}R^5 \qquad (XI)$$

in welcher

$R^5$ für Wasserstoff oder einen Acylrest steht, insbesondere für Wasserstoff, Acetyl, Propionyl, Benzoyl, 3-Chlorbenzoyl oder 4-Nitrobenzoyl steht,

gegebenenfalls in Gegenwart eines Verdünnungsmittels wie beispielsweise Aceton und gegebenenfalls in Gegenwart eines Katalysators wie beispielsweise Ammoniummolybdat bei Temperaturen zwischen -30 °C und +50 °C in allgemein üblicher Weise oxidiert zu den entsprechenden Sulfinyl- bzw. Sulfonylverbindungen der Formel (Vb),

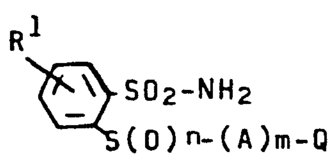

(Vb)

in welcher

$R^1$, A, Q und m die oben angegebene Bedeutung haben und

n für 1 oder 2 steht.

Verbindungen der Formel (Vc),

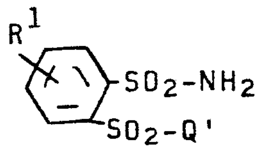

(Vc)

in welcher

$R^1$ die oben angegebene Bedeutung hat und

Q' für einen Heterocyclus der Formel

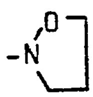 oder 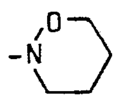 steht,

erhält man alternativ, wenn man 2-Chlorsulfonylphenylsulfonsäurefluoride der Formel (XII),

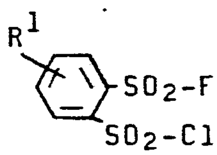

(XII)

in welcher

$R^1$ die oben angegebene Bedeutung hat,

zunächst in einer 1. Stufe mit Heterocyclen der Formel (XIII),

H-Q' (XIII)

in welcher

Q' die oben angegebene Bedeutung hat,

gegebenenfalls in Gegenwart eines Verdünnungsmittels wie beispielsweise Toluol und gegebenenfalls in Gegenwart eines Säurebindemittels wie beispielsweise Triethylamin bei Temperaturen zwischen -20 °C und +40 °C umsetzt, und die so erhaltenen Sulfonylfluoride der Formel (XIV),

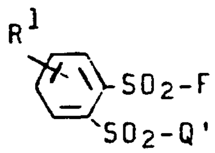

(XIV)

in welcher

$R^1$ und Q' die oben angegebene Bedeutung haben,

in einer 2. Stufe mit Ammoniak oder wäßrigem Ammoniumhydroxid bei Temperaturen zwischen +20 °C und +60 °C umsetzt.

Die zur Durchführung des erfindungsgemäßen Verfahrens (b - α) weiterhin als Ausgangsstoffe benötigten Iso-(thio-)cyanate sind durch die Formel (VI) allgemein definiert. In dieser Formel (VI) stehen Y und Z vorzugsweise für diejenigen Reste, die schon bei der Beschreibung der erfindungsgemäßen Stoffe der Formel (I) als bevorzugt für diese Substituenten genannt wurden.

Die Iso-(thio-)cyanate der Formel (VI) sind bekannt (vergl. z. B. EP 70 802, EP 70 804, EP 71 958, EP 72 347).

Die zur Durchführung des erfindungsgemäßen Verfahrens (b - β) weiterhin als Ausgangsstoffe benötigten (Thio-)Carbamate sind durch die Formel (VII) allgemein definiert. In dieser

Formel (VII) stehen Y und Z vorzugsweise für diejenigen Reste, die schon bei der Beschreibung der erfindungsgemäßen Stoffe der Formel (I) als bevorzugt für diese Substituenten genannt wurden. $R^{4'}$ steht vorzugsweise für Methyl, Ethyl oder Phenyl.

Die (Thio-)Carbamate der Formel (VII) sind bekannt oder lassen sich nach bekannten Methoden in einfachen Analogieverfahren herstellen (vergl. z. B. EP 70 802, EP 70 804, EP 71 958, EP 72 347, EP 79 683).

Die (Thio-)Carbonate bzw. Halogen-(thio-)ameisensäureester der Formel (VIII), die Sulfonamide der Formel (IX), die Halogenide der Formel (X), die Oxidationsmittel der Formel (XI), die Chlorsulfonylphenylsulfonsäurefluoride der Formel (XII) und die Heterocyclen der Formel (XIII) sind allgemein bekannte Verbindungen der organischen Chemie oder lassen sich nach allgemein bekannten Verfahren in analoger Weise herstellen (vergl. z. B. EP 19858, DE-OS 3 100 728).

Als Verdünnungsmittel zur Durchführung der erfindungsgemäßen Verfahren (a) (α und β) und (b) (α und β) kommen vorzugsweise inerte organische Lösungsmittel in Frage. Hierzu gehören insbesondere aliphatische oder aromatische, gegebenenfalls chlorierte Kohlenwasserstoffe wie beispielsweise Benzin, Benzol, Toluol, Xylol, Chlorbenzol, Petrolether, Hexan, Cyclohexan, Methylenchlorid, Chloroform, Tetrachlorkohlenstoff, Ether, wie Diethylether, Dioxan oder Tetrahydrofuran, Ketone wie Aceton oder Butanon, Nitrile wie Acetonitril oder Propionitril, Amide, wie Dimethylformamid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (a) (α und β) sowie (b)

(α und ß) können gegebenenfalls in Gegenwart eines Säurebindemittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat sowie tertiäre Amine, wie Triethylamin, N,N-Dimethylanilin, N,N-Dimethylaminopyridin, Pyridin, Diazabicyclononen (DBN) oder Diazabicycloundecen (DBU).

Die Reaktionstemperaturen können bei der Durchführung der erfingungsgemäßen Verfahren (a) (α und ß) und (b) (α und ß) in einem größeren Bereich variiert werden. Im allgemeinen arbeitet man bei Temperaturen zwischen -20 °C und +120 °C, vorzugsweise bei Temperaturen zwischen 0 °C und 50 °C.

Zur Durchführung des erfindungsgemäßen Verfahrens (a) setzt man pro Mol an heterocyclischer Aminoverbindung der Formel (II) im Fall der Variante (α) im allgemeinen 1.0 bis 1.5 Mol, vorzugsweise 1.0 bis 1.2 Mol an substituiertem Sulfonyliso-(thio-)cyanat der Formel (III) und im Fall der Variante (ß) im allgemeinen 1.0 bis 1.5 Mol, vorzugweise 1.0 bis 1.2 Mol an N-Sulfonyl-(thio-)carbamat der Formel (IV), sowie gegebenenfalls 1.0 bis 1.5 Mol an Säurebindemittel ein.

Zur Durchführung des erfindungsgemäßen Verfahrens (b) setzt man pro Mol an substituiertem Sulfonamid der Formel (V) im Fall der Variante (α) im allgemeinen 1 bis 1.5 Mol, vorzugsweise 1.0 bis 1.2 Mol an Iso-(thio-)cyanat der Formel (VI) und im Fall der Variante (ß) im allgemeinen 1.0 bis 1.5 Mol, vorzugsweise 1.0 bis 1.2 Mol an (Thio-)Carbamat der Formel (VII), sowie gegebenenfalls 1.0 bis 1.5 Mol an Säurebindemittel ein.

Die Aufarbeitung und Isolierung der Endprodukte der Formel (I) erfolgt bei allen Herstellungsverfahren in üblicher Art und Weise.

Die erfindungsgemäßen neuen substituierten Sulfonylharnstoffe (I) können als Pflanzenbehandlungsmittel verwendet werden. Unter "Pflanzenbehandlungsmittel" sind im Rahmen der vorliegenden Erfindung insbesondere herbizide, pflanzenwachstumsregulierende und fungizide Mittel zu verstehen. Im einzelnen gilt folgendes:

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfingungsgemäßen Wirkstoffe neben einer guten allgemein-herbiziden Wirkung gegenüber wichtigen Schadpflanzen auch eine gute Verträglichkeit gegenüber wichtigen Kulturpflanzen und können daher als Unkrautbekämpfungsmittel z. B. in Baumwolle oder Getreide eingesetzt werden.

Die erfindungsgemäßen Wirkstoffe greifen in den Metabolismus der Pflanzen ein und können deshalb auch als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch

wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung

sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfingungsgemäßen Wirkstoffe weisen darüberhinaus eine starke mikrobizide Wirkung auf und können in entsprechenden Aufwandmengen auch zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Reiskrankheiten wie beispielsweise gegen den Erreger der Reisfleckenkrankheit (Pyricularia oryzae) einsetzen.

Dabei zeigen die erfindungsgemäßen Wirkstoffe neben einer guten protektiven Wirksamkeit auch systemische Eigenschaften.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Schäume, Aerosole, Suspensions-Emulsions-Konzentrate, wirkstoffimprägnierte Natur- und synthetische Stoffe sowie Feinstverkapselungen in polymeren Stoffen und in Hüllmassen für Saatgut, sowie ULV-Formulierungen.

Diese Formulierungen werden in bekannter Weise hergestellt,

z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid.

Als feste Trägerstoffe kommen in Frage: z. B. natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Bei der Verwendung als Fungizide können die erfindungsgemäßen Wirkstoffe in den Formulierungen oder in den verschiedenen Anwendungsformen ebenfalls in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können dabei als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die nachfolgenden Beispiele dienen zur weiteren Erläuterung der Erfindung.

## Herstellungsbeispiele

Beispiel 1:

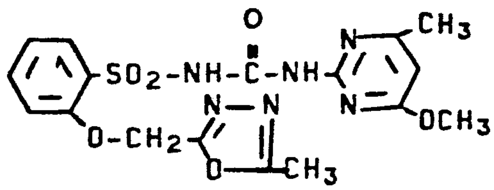

(1)

Zu einer Mischung von 5,4 g (0.02 Mol) 2-(5-Methyl-1,3,4-oxadiazol-2-yl-methoxy)-benzolsulfonamid, 5,2 g (0.02 Mol) O-Phenyl-N-(4-methoxy-6-methylpyrimidin-2-yl)-carbamat und 160 ml Acetonitril gibt man 3,2 g (0.02 Mol) Diazabicycloundecen (DBU) und rührt 12 Stunden bei Raumtemperatur. Zur Aufarbeitung gießt man den Reaktionsansatz in 700 ml Wasser, säuert mit Salzsäure an und extrahiert die wässrige Lösung mehrfach mit Essigester. Die vereinigten Essigesterphasen werden über Natriumsulfat getrocknet und im Vakuum eingeengt. Der Rückstand wird mit wenig Essigester verrieben und abgesaugt. Man erhält 5,1 g (59 % der Theorie) an N-[2-(5-Methyl-1,3,4-oxadiazol-2-yl-methoxy)-benzolsulfonyl]-N'-(4-methoxy-6-methyl-pyrimidin-2-yl)harnstoff vom Schmelzpunkt 165 °C - 168 °C.

Herstellung der Ausgangsverbindung:

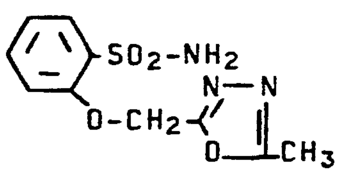

Zu einer Mischung aus 17,5 g (0.1 Mol) 2-Hydroxybenzolsulfonamid, 30 g (0.2 Mol) gepulvertem Kaliumcarbonat und 200 ml Acetonitril tropft man bei 70 °C unter Rühren 13,5 g (0.1 Mol) 2-Chlormethyl-5-methyl-1,3,4-oxadiazol. Man rührt weitere 12 Stunden bei 70 °C, filtriert den anorganischen Niederschlag

ab und engt das Filtrat im Vakuum ein. Der Rückstand wird mit Wasser verrieben und abgesaugt. Man erhält 13 g (48 % der Theorie) an 2-(5-Methyl-1,3,4-oxadiazol-2-ylmethyloxy)-benzolsulfonamid vom Schmelzpunkt 190 °C - 195 °C.

In entsprechender Weise und gemäß den allgemeinen Herstellungsangaben erhält man die folgenden Verbindungen der allgemeinen Formel (I)

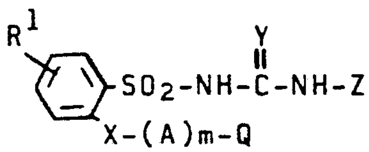

(I)

Tabelle 2

| Bsp.-Nr | $R^1$ | X | Y | Z | -(A)m- | Q | Schmelzpunkt/°C |
|---|---|---|---|---|---|---|---|
| 2 | - | O | O | 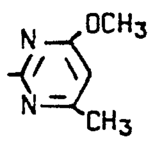 | $-CH_2-$ | 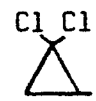 | 180 - 182 |
| 3 | - | O | O | 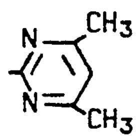 | $-CH_2-$ | 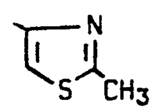 | 185 - 187 |
| 4 | - | O | O | 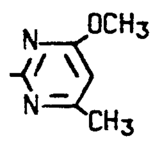 | $-CH_2-$ | 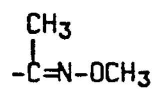 | 198 |
| 5 | - | O | O | 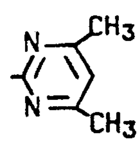 | $-CH_2-$ | 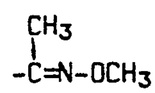 | 150 - 152 |
| 6 | - | O | O | 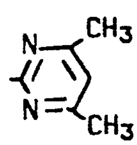 | $-CH_2-$ | 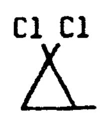 | 206 |
| 7 | - | O | O | 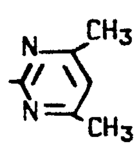 | $-CH_2-$ | 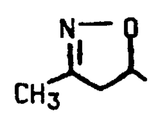 | 153 |

Tabelle 2 (Fortsetzung)

| Bsp.-Nr. | $R^1$ | X | Y | Z | -(A)m- | Q | Schmelzpunkt/°C |
|---|---|---|---|---|---|---|---|
| 8 | - | O | O | 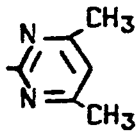 | $-CH_2-$ | 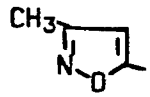 | 207 - 210 |
| 9 | - | O | O | 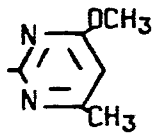 | $-CH_2-$ | 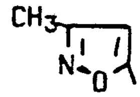 | 182 - 185 |
| 10 | - | O | O | 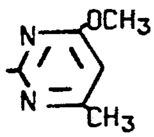 | $-CH_2-$ | 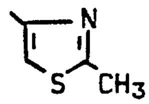 | 192 |
| 11 | - | O | O | 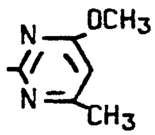 | $-CH_2-$ | $(CH_3)_3C-CO-$ | 209 |
| 12 | - | O | O | 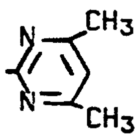 | $-CH_2-$ | $(CH_3)_3C-CO-$ | 212 - 214 |
| 13 | - | O | O | 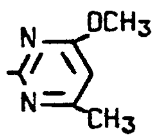 | - | 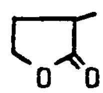 | 208 - 210 |
| 14 | - | O | O | 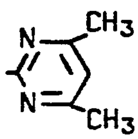 | - | 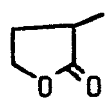 | 223 - 225 |
| 15 | - | O | O | 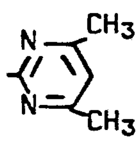 | $-CH_2-$ | $CH_3-CO-$ | 168 - 170 |
| 16 | - | O | O | 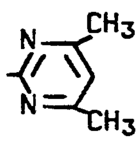 | $-CH_2-$ | 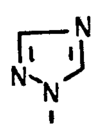 | 214 |
| 17 | - | O | O | 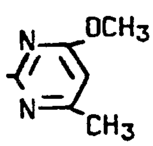 | $-CH_2-$ | 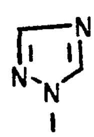 | 210 - 212 |

Tabelle 2 (Fortsetzung)

| Bsp.-Nr. | $R^1$ | X | Y | Z | -(A)m- | Q | Schmelzpunkt/°C |
|---|---|---|---|---|---|---|---|
| 18 | - | $SO_2$ | O | 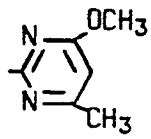 | - | 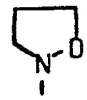 | 208 |
| 19 | - | $SO_2$ | O | 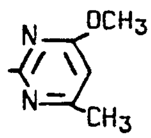 | - | 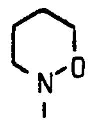 | 214 - 216 |
| 20 | - | $SO_2$ | O | 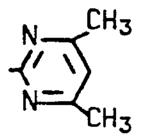 | - | 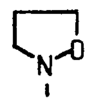 | 205 - 207 |
| 21 | 5-$NO_2$ | S | O | 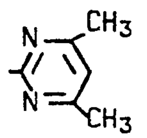 | - | 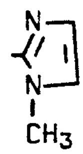 | 75 (Zers.) |
| 22 | - | S | O | 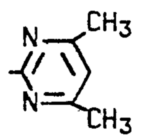 | - | 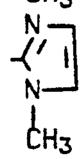 | 185 - 186 |
| 23 | 5-$NO_2$ | S | O | 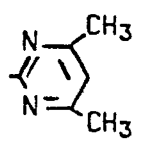 | $-CH_2-$ | 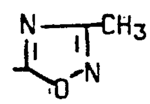 | 95 - 98 |
| 24 | 5-$NO_2$ | S | O | 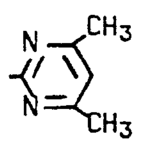 | $-CH_2-$ | 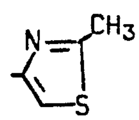 | 138 (Zers.) |
| 25 | 5-$NO_2$ | S | O | 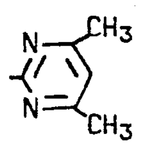 | $-CH_2-$ | 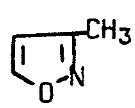 | 192 - 195 |
| 26 | 5-$NO_2$ | S | O | 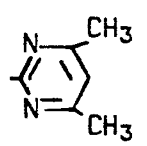 | - | 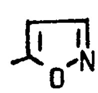 | 209 - 210 |

Anwendungsbeispiele

In den folgenden Anwendungsbeispielen werden die nachstehend aufgeführten Verbindungen als Vergleichssubstanzen herangezogen:

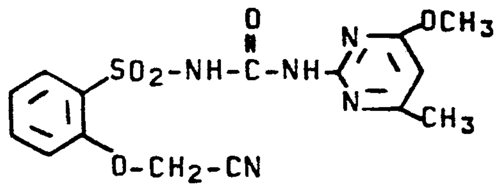

(A)

N-[(2-Cyanmethoxy)-phenylsulfonyl]-N'-(4-methoxy-6-methyl-pyrimidin-2-yl)-harnstoff

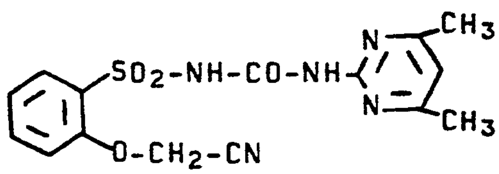

(B)

N-[(2-Cyanmethoxy)-phenylsulfonyl]-N'-(4,6-dimethyl-pyrimidin-2-yl)-harnstoff

(beide bekannt aus EP 85 028)

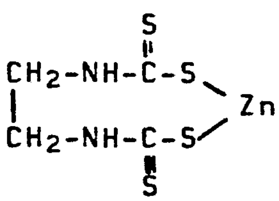

(C)

Zink-ethylen-1,2-bis(dithiocarbamat)

(bekannt z. B. aus R. Wegler 'Chemie der Pflanzenschutz- und Schädlingsbekämpfungsmittel', Springer Verlag Berlin 1970, Band 2, S. 65 f)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Ueberlegenheit in der herbiziden Wirkung und in der Nutzpflanzenselektivität gegenüber den aus dem Stand der Technik bekannten Verbindungen zeigen in diesem Test beispielsweise die Verbindungen gemäß den Herstellungsbeispielen: (2) und (10)

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Ueberlegenheit in der herbiziden Wirkung gegenüber den aus dem Stand der Technik bekannten Verbindungen zeigen in diesem Test beispielsweise die Verbindungen gemäß den Herstellungsbeispielen: (2) und (10)

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

Eine deutliche Ueberlegenheit in der Wirkung im Vergleich zur unbehandelten Kontrolle zeigt in diesem Test z. B. die Verbindung gemäß Herstellungsbeispiel: (9)

Beispiel D

Wuchshemmung bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Eine deutliche Ueberlegenheit in der Wirksamkeit im Vergleich zur unbehandelten Kontrolle zeigen in diesem Test z. B. die Verbindungen gemäß den Herstellungsbeispielen: 4, 5, 7, 9, 10, 15, 16, 17

Beispiel E

Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Eine deutliche Ueberlegenheit in der Wirkung im Vergleich zur unbehandelten Kontrolle zeigt in diesem Test z. B. die Verbindung gemäß Herstellungsbeispiel: (10)

Beispiel F

Wuchshemmung bei Gras (Festuca pratensis)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Eine deutliche Ueberlegenheit in der Wirksamkeit im Vergleich zur unbehandelten Kontrolle zeigen in diesem Test z. B. die Verbindungen gemäß den Herstellungsbeispielen: 4, 5, 7, 9, 16, 17.

Beispiel G

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß folgendem Herstellungsbeispiel : (6)

Beispiel H

Pyricularia-Test (Reis) / systemisch

Lösungsmittel: 12,5 Gewichtsteile Aceton
Emulgator: 0,3 Gewichtsteile Alkylarylpolykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß folgendem Herstellungsbeispiel : (6)

## Patentansprüche

1. Substituierte Sulfonylharnstoffe der allgemeinen Formel (I),

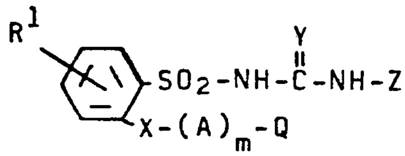

(I)

in welcher

$R^1$ für Wasserstoff, Halogen, Nitro, Alkyl, Alkoxy, Alkoxycarbonyl oder Halogenalkyl steht,

X für Sauerstoff, Schwefel, die Sulfinyl- oder die Sulfonylgruppe steht,

Y für Sauerstoff oder Schwefel steht,

Z für den Rest 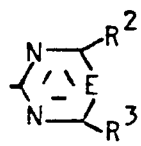 steht,

wobei E für Stickstoff oder die CH-Gruppe steht und $R^2$ und $R^3$ jeweils unabhängig voneinander für Alkyl, Alkoxy oder Alkoxyalkyl stehen,

A für einen zweifach verknüpften Alkylen- oder Alkenylenrest steht,

m für 0 oder 1 steht und

Q für Pivaloyl, für Methoximinoalkyl, für einen der Heterocyclen der Formel

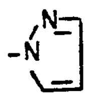 , 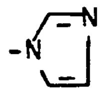 , 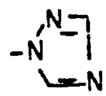 , 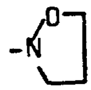 , 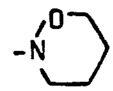 oder 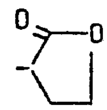

oder für einen der jeweils gegebenenfalls substituierten und/oder gegebenenfalls teilhydrierten C-verknüpften Heterocylen der Formel

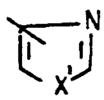 , 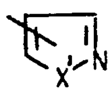 ; 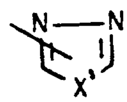 oder 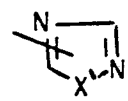 steht,

wobei X' jeweils für Sauerstoff, Schwefel oder eine NH-Gruppe steht,

außerdem für Acetyl steht, wenn $R^2$ und $R^3$ gleichzeitig für Alkyl stehen und weiterhin für 1,1-Dichlorcycloprop-2-yl steht, wenn E für die CH-Gruppe steht.

2. Substituierte Sulfonylharnstoffe der allgemeinen Formel (I) gemäß Anspruch 1, dadurch gekennzeichnet, daß darin

$R^1$ für Wasserstoff, Fluor, Chlor, Brom, Jod, Nitro, jeweils geradkettiges oder verzweigtes Alkyl, Alkoxy oder Alkoxycarbonyl mit jeweils bis zu 4 Kohlenstoffatomen in den einzelnen Alkylteilen oder für geradkettiges oder verzweigtes Halogenalkyl mit bis zu 4 Kohlenstoffatomen und bis zu 9 gleichen oder verschiedenen Halogenatomen steht,

X für Sauerstoff, Schwefel, die Sulfinyl- oder die Sulfonylgruppe steht,

Y für Sauerstoff oder Schwefel steht,

Z für den Rest 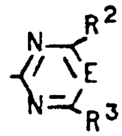 steht,

wobei E für Stickstoff oder die CH-Gruppe steht und
$R^2$ und $R^3$ jeweils unabhängig voneinander für jeweils geradkettiges oder verzweigtes Alkyl, Alkoxy oder Alkoxyalkyl mit bis zu 4 Kohlenstoffatomen in den einzelnen Alkylteilen stehen,

A für einen jeweils geradkettigen oder verzweigten zweifach verknüpften Alkylen- oder Alkenylenrest mit jeweils bis zu 4 Kohlenstoffatomen steht,

m für 0 oder 1 steht und

Q für Pivaloyl, für geradkettiges oder verzweigtes Methoximinoalkyl mit bis zu 4 Kohlenstoffatomen im Alkylteil, für einen der Heterocyclen der Formel

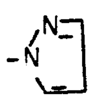 , 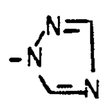 , 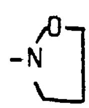 , 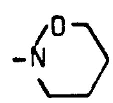 , 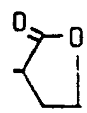 oder 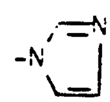

oder für einen der jeweils gegebenenfalls einfach oder mehrfach, gleich oder verschieden durch Halogen, Cyano, geradkettiges oder verzweigtes Alkyl oder Halogenalkyl mit jeweils bis zu 4 Kohlenstoffatomen und gegebenenfalls bis zu 9 gleichen oder verschiedenen Halogenatomen substituierten und/oder gegebenenfalls teilhydrierten C-verknüpften Heterocyclen der Formel

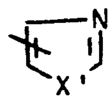 ; 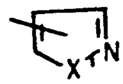 ; 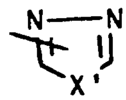 ; 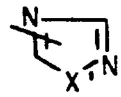 steht,

wobei X' jeweils für Sauerstoff, Schwefel oder die NH-Gruppe steht,

außerdem für Acetyl steht, wenn $R^2$ und $R^3$ gleichzeitig für Alkyl stehen und weiterhin für 1,1-Dichlorcycloprop-2-yl steht, wenn E für die CH-Gruppe steht.

3. Substituierte Sulfonylharnstoffe der allgemeinen Formel (I) gemäß Anspruch 1, dadurch gekennzeichnet, daß darin

$R^1$ für Wasserstoff, Fluor, Chlor, Brom, Nitro, Methyl, Ethyl, Methoxy, Ethoxy, Methoxycarbonyl, Ethoxycarbonyl, Trifluorethyl oder Trichlorethyl steht,

X für Sauerstoff, Schwefel, die Sulfinyl- oder die Sulfonylgruppe steht,

Y für Sauerstoff oder Schwefel steht,

Z für den Rest 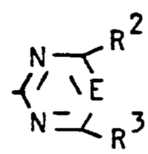 steht, wobei

E für Stickstoff oder die CH-Gruppe steht und

$R^2$ und $R^3$ unabängig voneinander für Methyl, Methoxy oder Methoxymethyl stehen,

A für $-CH_2-$; $-CH_2-CH_2-$; $\overset{CH_3}{-CH-}$; $\overset{CH_3}{-CH}-CH_2-$; $-CH=CH-$; $-CH_2-CH_2-CH_2-$ oder $\overset{CH_3}{-CH}-CH_2-CH_2-$ steht,

m für 0 oder 1 steht und

Q für Pivaloyl, 1-Methoximinoethyl, 1-Methoximinopropyl, 1-Methoximino-2-methylpropyl, für einen der Heterocyclen

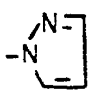 , 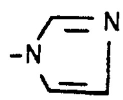 , 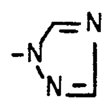 , 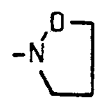 , 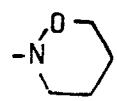 oder 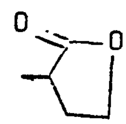

oder für einen jeweils gegebenenfalls einfach oder zweifach, gleich oder verschieden durch Methyl, Ethyl, Chlor, Brom, Cyano, Trifluormethyl, Trichlormethyl, Dichlorfluormethyl, Difluorchlormethyl, Chlormethyl oder Dichlormethyl substituierten Heterocyclus der Formel

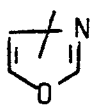 ; 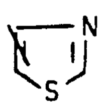 ; 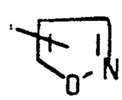 ; 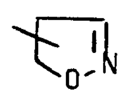 ; 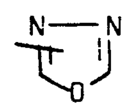 ; 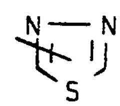 ;

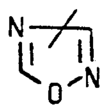 ; 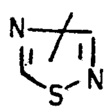 ; 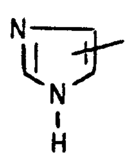 ; 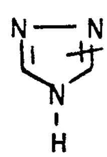 oder 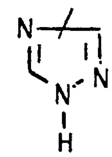 steht,

außerdem für Acetyl steht, wenn $R^2$ und $R^3$ gleichzeitig für Methyl stehen und weiterhin für 1,1-Dichlorcycloprop-2-yl steht, wenn E für die CH-Gruppe steht.

4. Verfahren zur Herstellung von substituierten Sulfonylharnstoffen der allgemeinen Formel (I) gemäß Anspruch 1, dadurch gekennzeichnet, daß man

(a) heterocyclische Aminoverbindungen der allgemeinen Formel (II),

$$H_2N-Z \qquad (II)$$

in welcher

Z die oben angegebene Bedeutung hat,

entweder

α) mit substituierten Sulfonyliso-(thio-)cyanaten der allgemeinen Formel (III)

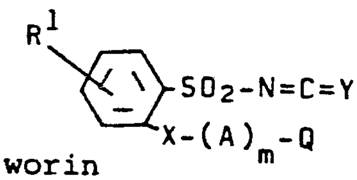

(III)

worin

$R^1$, X, Y, A, Q und m die oben angegebenen Bedeutungen haben,

oder

β) mit N-Sulfonyl-(thio-)-carbamaten der allgemeinen Formel (IV),

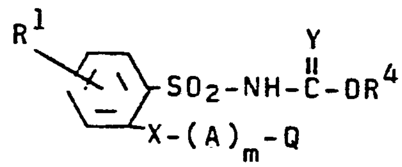

(IV)

in welcher

$R^1$, X, Y, A, Q und m die oben angegebene Bedeutung haben und

$R^4$ für Alkyl oder Aryl steht,

jeweils gegebenenfalls in Gegenwart eines Verdünnungsmittels und jeweils gegebenenfalls in Gegenwart eines Säurebindemittels umsetzt, oder daß man

(b) substituierte Sulfonamide der allgemeinen Formel (V),

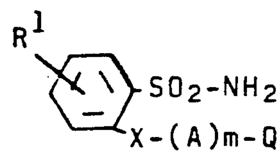

(V)

in welcher

$R^1$, X, A, Q und m die oben angegebene Bedeutung haben,

entweder

α) mit Iso-(thio)-cyanaten der Formel (VI),

Y=C=N-Z (VI)

in welcher

Y und Z die oben angegebene Bedeutung haben

oder

0160219

β) mit (Thio-) Carbamaten der Formel (VII),

$$R^{4'}-O-\overset{\overset{Y}{\|}}{C}-NH-Z \qquad (VII)$$

in welcher

Y und Z die oben angegebene Bedeutung haben und

$R^{4'}$ für Alkyl oder Aryl steht,

jeweils gegebenenfalls in Gegenwart eines Verdünnungsmittels und jeweils gegebenenfalls in Gegenwart eines Säurebindemittels umsetzt.

5. Pflanzenbehandlungsmittel, gekennzeichnet durch einen Gehalt an mindestens einem substituierten Sulfonylharnstoff der allgemeinen Formel (I) gemäß Anspruch 1.

6. Verwendung von substituierten Sulfonylharnstoffen der allgemeinen Formel (I) gemäß Anspruch 1 als Pflanzenbehandlungsmittel, insbesondere zur Bekämpfung von unerwüschtem Pflanzenwachstum, zur Regulierung des Pflanzenwachstums und zur Bekämpfung von unerwünschten Mikroorganismen, jeweils in geeigneten Konzentrationen bzw. Aufwandmengen.

7. Verfahren zur Herstellung von Pflanzenbehandlungsmitteln, dadurch gekennzeichnet, daß man substituierte Sulfonylharnstoffe der allgemeinen Formel (I) gemäß Anspruch 1 mit Streckmitteln und/oder oberflächenaktiven Mitteln vermischt.

Europäisches Patentamt

**EUROPÄISCHER RECHERCHENBERICHT**

0160219
Nummer der Anmeldung

EP 85103859.6

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| D,A | US - A - 4 127 405 (LEVITT)<br>* Ansprüche 1,27 *<br>-- | 1,5 | C 07 D 251/16<br>C 07 D 251/46<br>C 07 D 251/22<br>C 07 D 239/42<br>C 07 D 239/47<br>C 07 D 403/12<br>C 07 D 405/12<br>C 07 D 413/12<br>A 01 N 47/34<br>A 01 N 47/36 |
| A | US - A - 4 435 206 (LEVITT)<br>* Ansprüche 1,16 *<br>-- | 1,5 | |
| A | EP - A2/A3 - 0 094 790 (DU PONT)<br>* Zusammenfassung *<br>-- | 1,5 | |
| D,A | EP - A1 - 0 072 347 (CIBA)<br>* Zusammenfassung *<br>---- | 1,5 | |
| | | | RECHERCHIERTE SACHGEBIETE (Int. Cl.4) |
| | | | C 07 D 251/00<br>C 07 D 239/00<br>C 07 D 403/00<br>C 07 D 405/00<br>C 07 D 413/00 |

Der vorliegende Recherchenbericht wurde für alle Patentansprüche erstellt.

| Recherchenort | Abschlußdatum der Recherche | Prufer |
|---|---|---|
| WIEN | 18-06-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82